# EUROPEAN PATENT APPLICATION

(11) **EP 1 864 664 A1**
(43) Date of publication of application: **12.12.2007**
(21) Application number: 06715510.1
(22) Date of filing: 10.03.2006
(51) Int. Cl.: A61K 31/501, A61K 9/08, A61K 9/19, A61K 47/10, A61K 47/12, A61K 47/26, A61K 47/32, A61K 47/34, A61P 9/04, A61P 9/12, A61P 27/02, A61P 43/00

(54) **PHARMACEUTICAL PREPARATION**

(30) Priority: 10.03.2005 JP 2005066521
(71) Applicant: Mitsubishi Pharma Corporation, Osaka-shi, Osaka 541-0046 (JP)
(72) Inventor: OZAKI, Kazuhiko, MITSUBISHI PHARMA CORPORATION, Chuo-ku , Tokyo, 103-8405 (JP); HIRAMIYA, Manami, MITSUBISHI PHARMA CORPORATION, Chuo-ku , Tokyo, 103-8405 (JP)
(74) Representative: Merkle, Gebhard
(86) International application number: PCT/JP2006/304690
(87) International publication number: WO 2006/095844

(57) **Abstract**

Pharmaceutical products comprising 6-[4-(4-pyridylamino)phenyl]-4,5-dihydro-3(2H)-pyridazinone or a salt thereof, or a hydrate thereof or a solvate thereof as an active ingredient, wherein production of hydrazine is substantially suppressed is provided.

## Description

### Technical Field

The present invention relates to a pharmaceutical products containing a specific phenylpyridazinone derivative as an active ingredient.

### Background Art

6-[4-(4-Pyridylamino)phenyl]-4,5-dihydro-3(2H)-pyridazinone is known to have a superior cardiotonic action, and be a useful compound as a medicament (Patent documents 1, 2 and 3). However, to the best knowledge of the inventors of the present invention, no report has been made so far as for the above compound about what pharmaceutical formulations should be prepared when used as a solution, for example, used as an injection.
Patent Document 1: Japanese Patent Unexamined Publication (Kokai) No. 60-126282
Patent Document 2: Japanese Patent Unexamined Publication No. 61-289032
Patent Document 3: International Patent Publication WO02/088109

### Disclosure of the Invention

### Object to be Achieved by the Invention

An object of the present invention is to provide a novel pharmaceutical form of a medicament comprising a specific phenylpyridazinone derivative as an active ingredient.

### Means for Achieving the Object

The inventors of the present invention conducted various researches to achieve the foregoing object. As a result, they found a stable pharmaceutical form that can be provided as, for example, an injection. The present invention was achieved on the basis of the above finding.

The gists of the present invention are as follows:
(1) Pharmaceutical products comprising 6-[4-(4-pyridylamino)phenyl]-4,5-dihydro-3(2H)-pyridazinone or a salt thereof, or a hydrate thereof or a solvate thereof as an active ingredient, provided that an aqueous solution is excluded, wherein production of hydrazine is substantially suppressed.
(2) The pharmaceutical products according to (1), wherein the active ingredient is 6-[4-(4-pyridylamino)phenyl]-4,5-dihydro-3(2H)-pyridazinone hydrochloride trihydrate.
(3) The pharmaceutical products according to (1) or (2), wherein an amount of the hydrazine produced after storage of the pharmaceutical products at 60°C for 30 days is less than about 0.23% (w/w) based on the active ingredient.
(4) The pharmaceutical products according to (1) or (2), wherein an amount of the hydrazine produced after storage of the pharmaceutical products at 60°C for 30 days is not more than about 0.05% (w/w) based on the active ingredient.
(5) The pharmaceutical products according to any one of (1) to (4), wherein the active ingredient is dissolved in a non-aqueous solvent.
(6) The pharmaceutical products according to (5), wherein the non-aqueous solvent consists of one or more types of solvents selected from glycerin, propylene glycol, polysorbate, ethanol and polyethylene glycol.
(7) The pharmaceutical products according to any one of (1) to (4), which are lyophilized products.
(8) The pharmaceutical products according to (7), which contain a saccharide.
(9) The pharmaceutical products according to (7) or (8), which contain a pH stabilizer.
(10) The pharmaceutical products according to any one of (7) to (9), which are dissolved upon use.
(11) Pharmaceutical products comprising 6-[4-(4-pyridylamino)phenyl]-4,5-dihydro-3(2H)-pyridazinone or a salt thereof, or a hydrate thereof or a solvate thereof as an active ingredient, wherein an amount of hydrazine produced after storage of the pharmaceutical products at 60°C for 30 days is less than about 0.23% (w/w) based on the active ingredient.
(12) The pharmaceutical products according to (11), wherein the active ingredient is 6-[4-(4-pyridylamino)phenyl]-4,5-dihydro-3(2H)-pyridazinone hydrochloride trihydrate.
(13) The pharmaceutical products according to (11) or (12), wherein the amount of the hydrazine produced is not more than about 0.05% (w/w) based on the active ingredient.
(14) The pharmaceutical products according to any one of (11) to (13), which are non-aqueous solution products, or lyophilized products.
(15) The pharmaceutical products according to (14), wherein a non-aqueous solvent of the non-aqueous solution products consist of one or more types of solvents selected from glycerin, propylene glycol, polysorbate, ethanol and polyethylene glycol.
(16) The pharmaceutical products according to (14), wherein the lyophilized products contain a saccharide.
(17) The pharmaceutical products according to (14) or (16), wherein the lyophilized products contain a pH stabilizer.
(18) The pharmaceutical products according to any one of (14), (16) or (17), wherein the lyophilized products are dissolved upon use.
(19) A method for stabilization of a solution containing 6-[4-(4-pyridylamino)phenyl]-4,5-dihydro-3(2H)-pyridazinone or a salt thereof, or a hydrate thereof or a solvate thereof, which comprises the step of adjusting a pH of the solution within a range of from about 3 to about 7.
(20) The method for stabilization according to (19), wherein the solution contains a pH stabilizer.
(21) The method for stabilization according to (19) or (20), wherein the solution contains an isotonic agent.

### Effect of the Invention

According to the present invention, a novel pharmaceutical form of 6-[4-(4-pyridylamino)phenyl]-4,5-dihydro-3(2H)-pyridazinone useful as a cardiotonic agent can be provided.

### Brief Description of the Drawings

[Fig. 1] A graph depicting the influence of a pH on changes in a content of the compound.
[Fig. 2] A graph depicting changes in pH over time observed for Preparation Example 7.
[Fig. 3] A graph depicting changes in pH over time observed for Preparation Example 8.
[Fig. 4] A graph depicting changes in pH over time observed for Preparation Example 11.
[Fig. 5] A graph depicting changes in pH over time observed for Preparation Example 12.
[Fig. 6] A graph depicting changes in pH over time observed for Preparation Example 9.
[Fig. 7] A graph depicting changes in pH over time observed for Preparation Example 10.
[Fig. 8] A graph depicting changes in pH over time observed for Preparation Example 13.
[Fig. 9] A graph depicting changes in pH over time observed for Preparation Example 14.
[Fig. 10] A graph depicting changes in pH over time observed for Preparation Example 1.
[Fig. 11] A graph depicting changes in pH over time observed for Preparation Example 2.
[Fig. 12] A graph depicting changes in pH over time observed for Preparation Example 3.
[Fig. 13] A graph depicting changes in pH over time observed for Preparation Example 4.
[Fig. 14] A graph depicting changes in pH over time observed for Preparation Example 5.
[Fig. 15] A graph depicting changes in pH over time observed for Preparation Example 6.

### Best Mode for Carrying out the Invention

The present invention will be explained in detail below.

6-[4-(4-Pyridylamino)phenyl]-4,5-dihydro-3(2H)-pyridazinone or salts thereof, or hydrates thereof and solvates thereof are compounds described in Japanese Patent Unexamined Publication (Kokai) Nos. 58-8015, 58-8016, 58-140076 and 60-126282, Journal of Medicinal Chemistry, 1987, vol. 30, No. 7, 1157-1161, and International Publication WO02/088109, and can be prepared according to the methods described in these publications or similar methods thereto.

Salts thereof are not particularly limited so long as the salts are pharmaceutically acceptable. Examples thereof include salts with mineral acids such as hydrochloric acid and phosphoric acid, and salts with organic acids such as lactic acid and acetic acid.

According to the present invention, the aforementioned compounds can be used as hydrates or solvates. The solvates are not particularly limited so long as solvents are pharmaceutically acceptable.

According to the present invention, the most preferred compound is 6-[4-(4-pyridylamino)phenyl]-4,5-dihydro-3(2H)-pyridazinone hydrochloride trihydrate.

The aforementioned compounds produce hydrazine by hydrolysis.

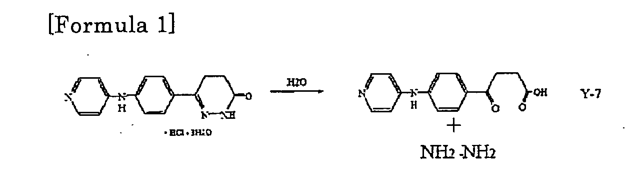

Since hydrazine is a carcinogenic substance, it is desirable to suppress the production thereof as far as possible for uses as a medicament.

The present invention provides pharmaceutical products in which the aforementioned production of hydrazine is substantially suppressed. According to the present invention, it is sufficient that production of hydrazine is suppressed to be within the pharmaceutically acceptable range, and the degree of the suppression is not particularly limited. For example, in a storage stability test at 60°C for 30 days as described in Example 4 mentioned later, an amount of the production of hydrazine is, for example, less than about 0.23% (w/w), preferably not more than about 0.05% (w/w), based on the amount of the active ingredient. The amount of the production of hydrazine is assumed when 2 mg of the aforementioned active ingredient per day is administered via an intravenous drip infusion over 60 minutes for 5 days at the maximum. Therefore, the amount of the production of hydrazine according to the present invention can be appropriately increased or decreased depending on the dose and administration method by referring as a standard to the aforementioned amount of the production of hydrazine given as an example.

Hydrazine is produced by hydrolysis of the compound as the active ingredient of the present invention as described above. Accordingly, for obtaining a desired pharmaceutical products according to the present invention, preferred examples of the pharmaceutical products include pharmaceutical products obtained by dissolving the active ingredient in a non-aqueous solvent. The non-aqueous solvent is not particularly limited so long as the solvent is pharmaceutically acceptable, and examples include, for example, one or more types of solvents selected from glycerin, propylene glycol, polysorbate, ethanol and polyethylene glycol.

The pharmaceutical products of the present invention can also be provided in the form of lyophilized products. The lyophilized products can be produced by, for example, preparing a compound as the active ingredient of the present invention as an aqueous solution containing a saccharide, adding a suitable additive, if necessary, and lyophilizing the solution in a conventional manner. The saccharide to be used is not particularly limited, and sucrose, mannitol, and the like can be used.

A further aspect of the present invention includes a method for stabilizing a solution containing the aforementioned compound. For this purpose, a pH of the solution is adjusted in a range of from about 3 to about 7. If a pH is lower than this range, 6-[4-(4-pyridylamino)phenyl]-4,5-dihydro-3(2H)-pyridazinone or a salt thereof, or a hydrate thereof or a solvate thereof is unstable. Further, if a pH is in an alkaline range, crystallization is observed. The pH is preferably in a range of from about 5 to 6, more preferably about 5.5. As a pH adjustment agent, an appropriate agent may be used for obtaining the pH in the aforementioned range, and sodium hydroxide and the like can be used.

In the stabilization method of the present invention, it is preferable to add an appropriate pH stabilizer to the solution. Examples of the pH stabilizer include citric acid, phosphoric acid, tartaric acid, lactic acid, and the like, and citric acid is preferred.

An amount of the pH stabilizer is suitably determined depending on the pH stabilizer to be used and the amount of 6-[4-(4-pyridylamino)phenyl]-4,5-dihydro-3(2H)-pyridazinone or a salt thereof, or a hydrate thereof or a solvate thereof contained in the products as shown in the examples described later. For example, when citric acid is used as the pH stabilizer, a preferred amount is about 1 mM.

In the stabilization method of the present invention, an isotonic agent can be optionally added, and preferred examples of the isotonic agent include D-sorbitol, and the like.

Example of the method for preparing a solution in the stabilization method of the present invention include the following method. Specifically, an isotonic agent, a pH stabilizer and distilled water for injection are added to 6-[4-(4-pyridylamino)phenyl]-4,5-dihydro-3(2H)-pyridazinone or a salt thereof, or a hydrate thereof or a solvate thereof and are dissolved at room temperature, and then a pH adjustment agent is added dropwise with stirring to adjust the pH. Distilled water for injection is then added to adjust the total volume.

When the pharmaceutical products of the present invention are clinically used, 0.01 to 200 mg of the aforementioned compound may be preferably administered 2 to 5 times a day in the case of intravenous injection, or the dose as mentioned above may be continuously administered by drip infusion. For use as an eye drop, 0.1 to 100 mg/ml of the aforementioned compound may be preferably instilled as 1 or 2 drops once to several times a day. The aforementioned compound can also be used by addition to a solution for intraocular perfusion. The aforementioned doses may be appropriately increased or decreased depending on age, pathological conditions, sexuality, symptoms, and the like.

When the pharmaceutical products of the present invention are used as an intravenous injection, the products may also be used in the form of a sterilized injection such as those contained in an ampoule or an infusion bag.

In the case of eye drop, thickeners, suspending agents, emulsifiers, preservatives, and the like may be optionally added, if necessary.

When the pharmaceutical products of the present invention are provided as lyophilized products, the products are appropriately dissolved upon use.

The pharmaceutical products of the present invention can be used as an anti-heart failure agent, antihypertensive agent, calcium ion sensitivity enhancer, cardiotonic agent, agent for ophthalmology, or the like.

### Examples

The present invention will be explained in more detail with reference to the following examples. However, the present invention is not limited to these examples unless they are beyond the gist of the invention. The compound used in the following examples was 6-[4-(4-pyridylamino)phenyl]-4,5-dihydro-3(2H)-pyridazinone hydrochloride trihydrate, and the compound prepared according to the method described in International Patent Publication WO02/08819 was used.

### Example 1

A solution was prepared so as to contain the compound at 1 mg/mL of a solvent, and divided as aliquots in clear test tubes and the tubes were tightly sealed. The Britton-Robinson buffer was used as the solvent to prepare sample solutions at pH 2, 3, 4, 5 and 6.

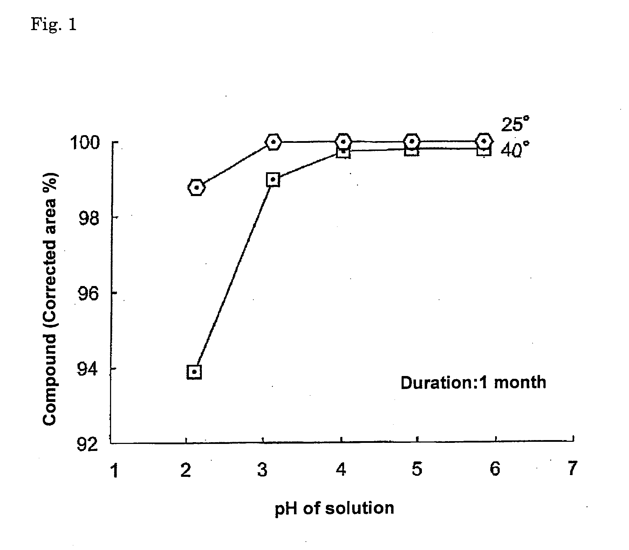

After 1 month storage of these sample solutions under each temperature condition of 25 or 40°C, the contents of the compound were determined by HPLC under the following measurement conditions.
Sample solution: 1 mL of specimen/25 mL of mobile phase
Column: Nucleosil 5C₁₈ 4.6 x 250 mm
Precolumn: Nucleosil 10C₁₈ 4.6 x 10 mm
Temperature: Room temperature
Mobile phase: 1 M Et₃N (adjusted at pH 8 with AcOH):CH₃CN:H₂O = 50:250:700
Flow rate: 1.2 mL/sec
Detection: UV 340 nm
Loaded volume : 20 µL
Quantification method: Corrected area percentage
The results are shown in Fig. 1. It can be understood that the remaining ratio of the compound becomes highest at pH 5 to 6.

### Preparation Examples 1 to 14

Pharmaceutical products were obtained with the following formulations. For all of the products, to the compound was added D-sorbitol as the isotonic agent, citric acid, phosphoric acid, or tartaric acid as the pH stabilizer, and distilled water for injection, the compound was dissolved at room temperature, and then NaOH as the pH adjustment agent was added dropwise to the solution with stirring to adjust the pH. Then, distilled water for injection was added to adjust the total volume. Each product was then divided in ampoules, which were subjected to ultrasonic cleaning and dry sterilization at 250°C for 30 minutes beforehand, and then sealed by melt welding. Before and after the filling, the gaseous phase was replaced with nitrogen. Then, the ampoules were subjected to steam sterilization at 121°C for 20 minutes.

**Table 1: Examples of products containing 0.5 mg of compound/mL**

| | Preparation Example 1 | Preparation Example 2 | Preparation Example 3 | Preparation Example 4 | Preparation Example 5 | Preparation Example 6 |
|---|---|---|---|---|---|---|
| MCI-154 (mg) | 0.5 | 0.5 | 0.5 | 0.5 | 0.5 | 0.5 |
| D-Sorbitol (mg) | 270 | 270 | 250 | 250 | 240 | 240 |
| Citric acid (mg) | 0 | 10.5 | 0.21 | 0.53 | 0 | 0 |
| Phosphoric acid (mg) | 0 | 0 | 0 | 0 | 2.88 | 0 |
| Tartaric acid (mg) | 0 | 0 | 0 | 0 | 0 | 3.76 |
| NaOH | Suitable amount | Suitable amount | Suitable amount | Suitable amount | Suitable amount | Suitable amount |
| Distilled water for injection (total volume mg) | 5 | 5 | 5 | 5 | 5 | 5 |
| pH before sterilization | 5.46 | 5.61 | 5.61 | 5.6 | 5.62 | 5.59 |
| pH after sterilization | 6 | 5.64 | 5.72 | 5.58 | 5.6 | 5.58 |

| | | | | | | |
|---|---|---|---|---|---|---|
| Note: Preparation Example 1: no pH stabilizer, Preparation Example 2: 10 mM citric acid, Preparation Example 3: 0.2 mM citric acid, Preparation Example 4: 0.5 mM citric acid, Preparation Example 5: 5 mM phosphoric acid, Preparation Example 6: 5 mM tartaric acid. | | | | | | |

**Table 2: Examples of products containing 5 mg of compound/5 mL**

| | Preparation Example 7 | Preparation Example 8 | Preparation Example 9 | Preparation Example 10 | Preparation Example 11 | Preparation Example 12 | Preparation Example 13 | Preparation Example 14 |
|---|---|---|---|---|---|---|---|---|
| MCI-154 (mg) | 5 | 5 | 5 | 5 | 5 | 5 | 5 | 5 |
| D-Sorbitol (mg) | 270 | 270 | 270 | 270 | 254 | 245 | 240 | 240 |
| Citric acid (mg) | 0 | 10.5 | 10.5 | 10.5 | 1.05 | 2.1 | 0 | 0 |
| Phosphoric acid (mg) | 0 | 0 | 0 | 0 | 0 | 0 | 2.88 | 0 |
| Tartaric acid (mg) | 0 | 0 | 0 | 0 | 0 | 0 | 0 | 3.76 |
| NaOH | Suitable amount | Suitable amount | Suitable amount | Suitable amount | Suitable amount | Suitable amount | Suitable amount | Suitable amount |
| Distilled water for injection (total volume mg) | 5 | 5 | 5 | 5 | 5 | 5 | 5 | 5 |
| pH before sterilization | 5.48 | 5.48 | 4.97 | 5.98 | 5.5 | 5.5 | 5.57 | 5.53 |
| pH after sterilization | 5.81 | 5.47 | 5 | 5.99 | 5.51 | 5.51 | 5.53 | 5.55 |

| | | | | | | | | |
|---|---|---|---|---|---|---|---|---|
| Note: Preparation Example 7: no pH stabilizer, Preparation Examples 8 to 10: 10 mM citric acid, Preparation Example 11: 1 mM citric acid, Preparation Example 12: 2 mM citric acid, Preparation Example 13: 5 mM phosphoric acid, Preparation Example 14: 5 mM tartaric acid. | | | | | | | | |

### Example 2

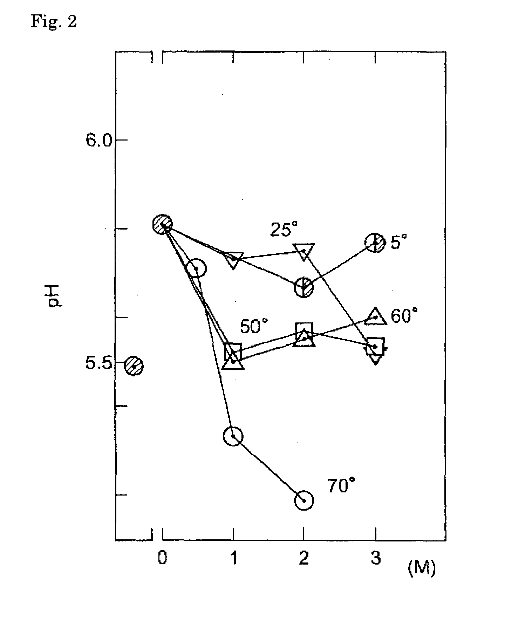

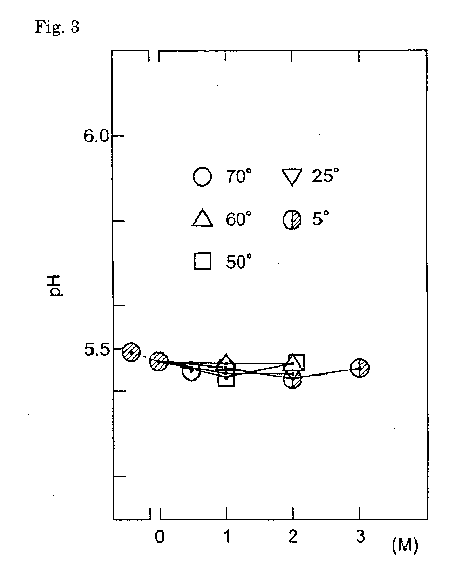

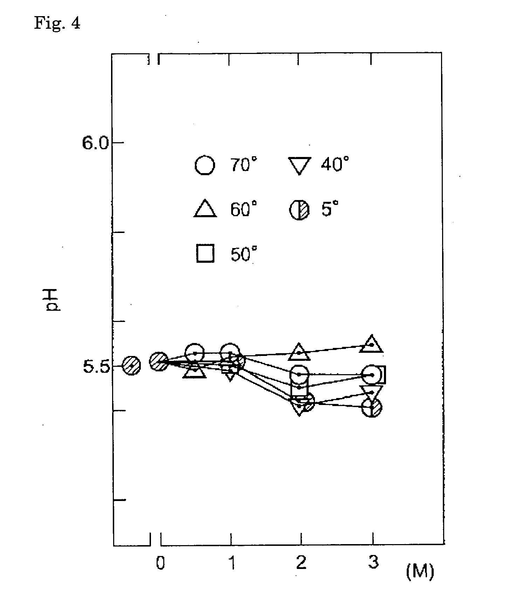

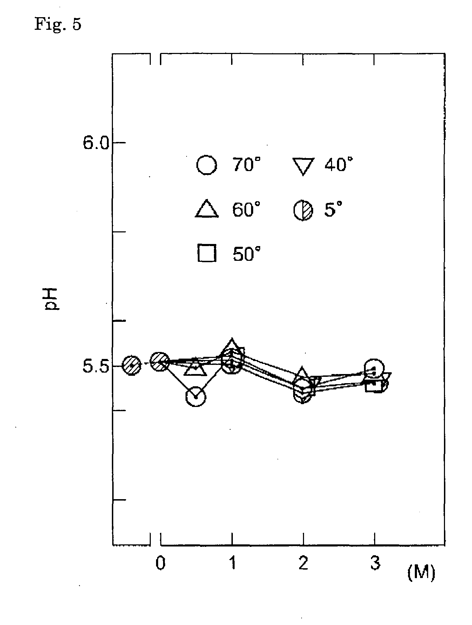

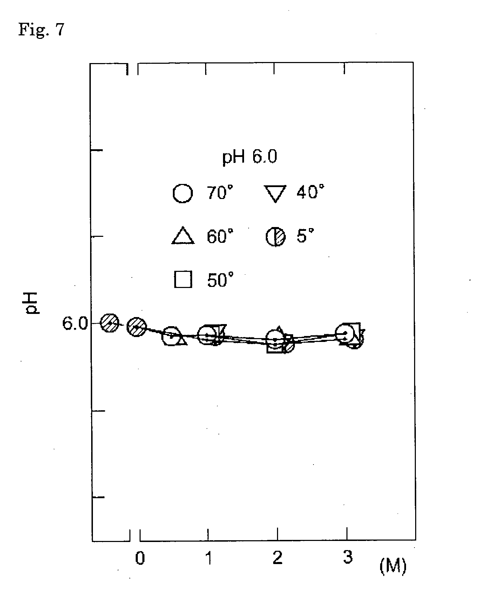

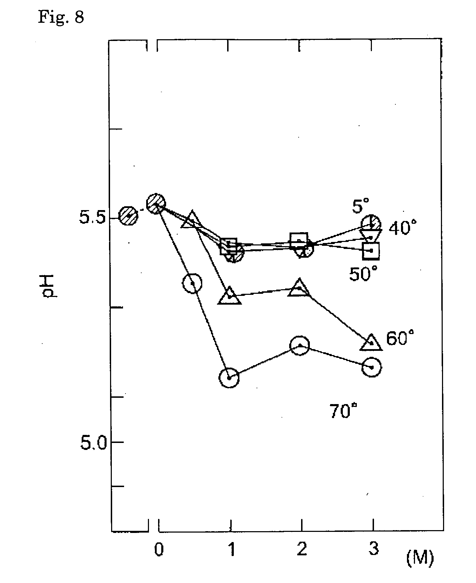

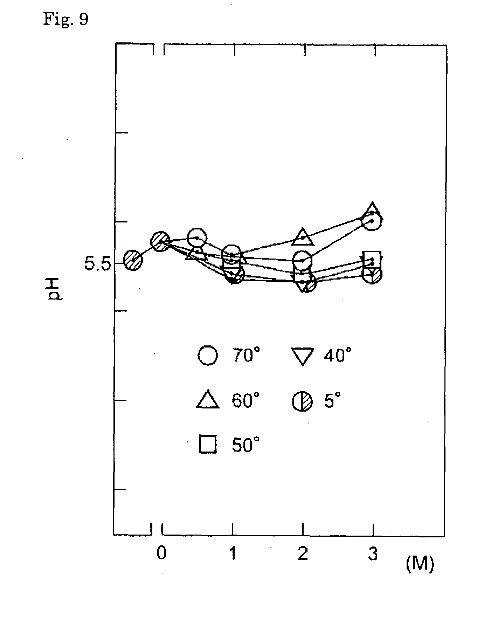

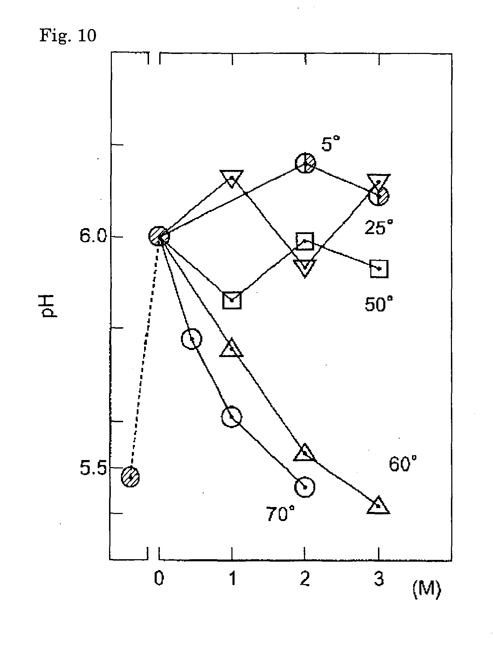

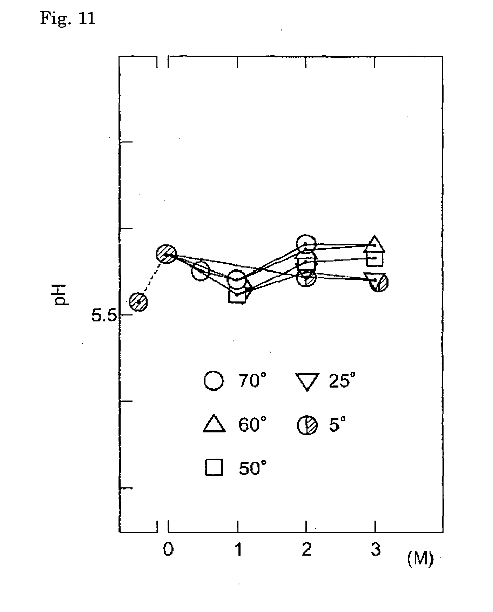

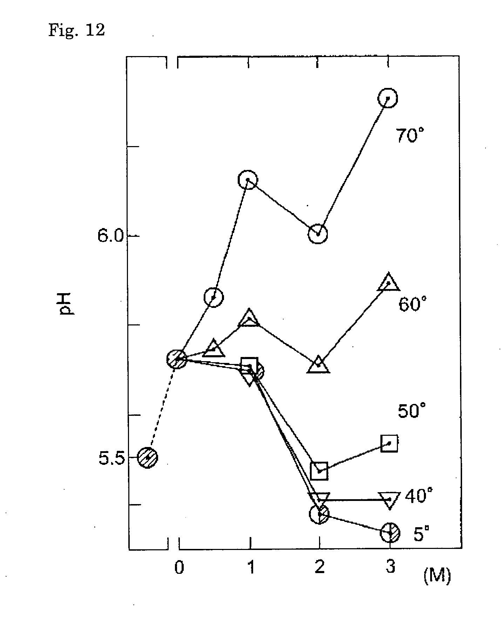

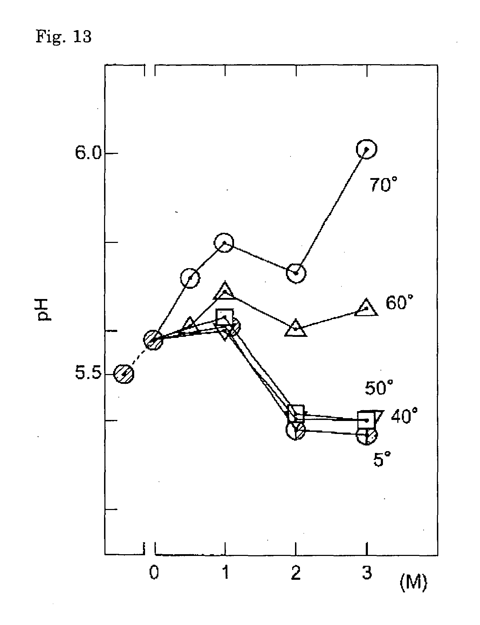

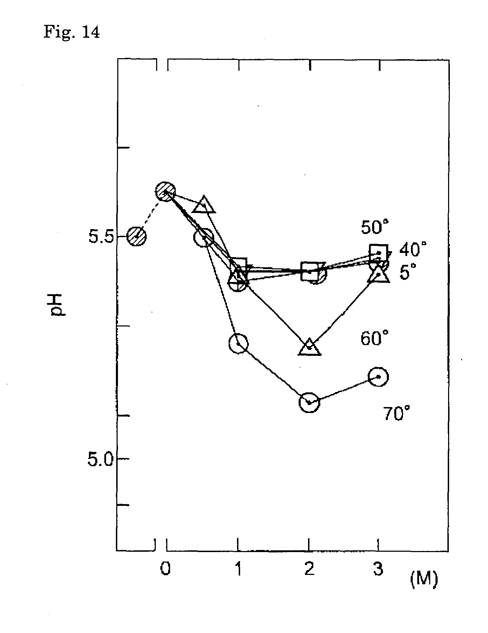

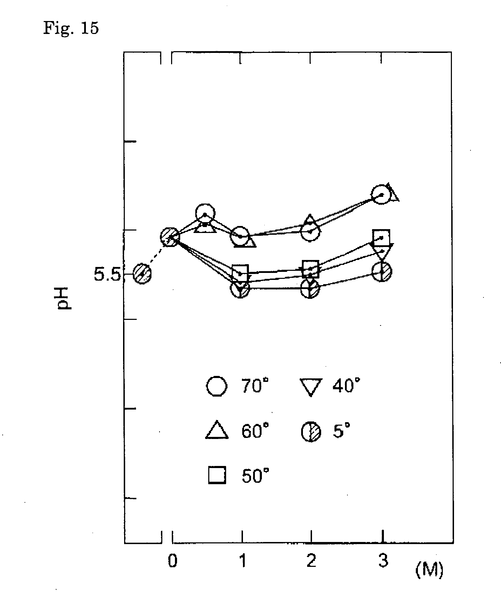

The products obtained according to the aforementioned preparation examples were stored at 70, 60, 50, 25 or 5°C for 0.5, 1, 2 or 3 months, and changes in a pH of the products over time were examined. The results are shown in Figs. 2 to 15.

Figs. 2 to 5 and Figs. 10 to 13 show the results of examination of pH stability of the formulated solutions performed by changing the concentration of citric acid as the pH stabilizer. From these results, it can be understood that, when citric acid is not added, the pH significantly changes after the steam sterilization, and the pH of the formulated solution was unstable. In the formulation of 5 mg/5 mL, almost no change in the pH after the steam sterilization was observed by the addition of citric acid, and the pH stability of the formulated solution was also improved. In the formulation of 0.5 mg/5 mg, the pH of the formulated solution became unstable even after the addition of 0.2 or 0.5 mM citric acid. These results suggest that 1 mM is sufficient as the concentration of citric acid required for the pH stability.

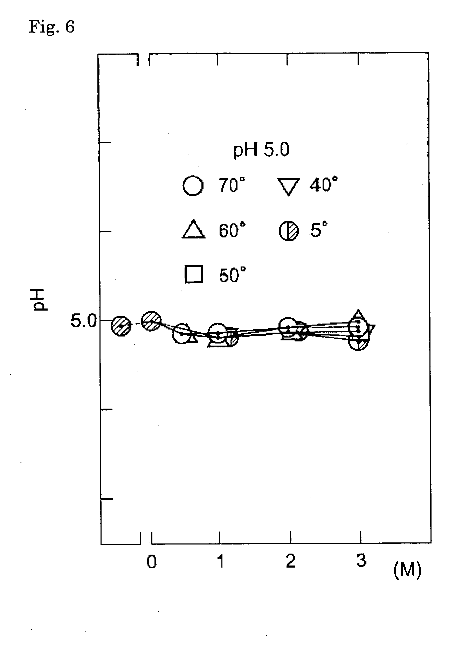

The results of the examination of the pH stability of the formulated solutions, performed by changing the pH of the formulated solution at the time of preparation and the pH stabilizer used, are shown in Figs. 6 to 9 and Figs. 14 to 15.

### Example 3

Lyophilized products were prepared with the formulations for solution and lyophilization conditions shown in Tables 3 and 4 mentioned below. It was confirmed that each cake shape was favorable and no problem of resolubility occurred in each formulation shown below.

**Table 3: Formulations for solution (amounts of components per vial)**

| Component | Preparation Example 15 | Preparation Example 16 |
|---|---|---|
| Compound | 5 mg | 5 mg |
| Sucrose | 200 mg | - |
| D-Mannitol | - | 100 mg |
| Citric acid | 0.84 mg | 0.84 mg |
| Sodium hydroxide | Suitable amount | Suitable amount |
| Water for injection | Suitable volume | Suitable volume |
| Total volume | 2 mL | 2 mL |

**Table 4: Lyophilization conditions**

| | | | | | | |
|---|---|---|---|---|---|---|
| Temperature (°C) | 20→40 | -40 | -40→20 | -20 | -20→20 | 20 |
| Time (minute) | 60 | 60 | 120 | 840 | 240 | 60 |
| | | | → Vacuuming started | | | |

### Example 4

Pharmaceutical products were obtained with the following formulations and stored in vials at 60°C for 30 days.

**Table 5: Formulations for solution**

| | Solvent | Compound (mg) | Total volume (mL) | Final concentration of compound (mg/mL) |
|---|---|---|---|---|
| Preparation Example 17 | Glycerin | 2502.0 | 50 | 50 |
| Preparation Example 18 | 50:50 (w/w) glycerin/propylene glycol | 2500.7 | 50 | 50 |
| Preparation Example 19 | 50:50 (w/w) polysorbate 80/ethanol | 200.6 | 50 | 4 |
| Preparation Example 20 | 50:50 (w/w) Polyethylene glycol 300/ethanol | 201.0 | 50 | 4 |
| Preparation Example 21 | 50 mM phosphate buffer, pH 7 | 10.5 | 50 | 0.2 |

Then, each amount of Y-7 produced in each solution was measured by HPLC under the following measurement conditions. Sample solution: 1 mL of specimen/25 mL of mobile phase
Column: Waters Symmetry C18, 3.9 x 150 mm, 5 µm, Part# WAT046980, Lot# W92361
Eluent A: 58.8 mM KPO₄, pH 4.7
Eluent B: Methanol

**Pump Gradient Program**

| Time (minute) | %A | %B |
|---|---|---|
| 0.01 | 80 | 20 |
| 8.00 | 80 | 20 |

Flow rate: 1.0 mL/sec
Column temperature: 38°C
Loaded volume: 10 µL,
Sample temperature: 10°C
Detection: 254 nm
Operation time: 8 minutes
Sample diluent: 58.8 mM KPO₄, pH 4.7
The results are shown below. The "%" mentioned below represents a ratio of the area of Y-7 peaks, and RRT means relative retention time.

**Table 6**

| Preparation Example 17 | | Preparation Example 18 | | Preparation Example 19 | | Preparation Example 20 | | Preparation Example 21 | |
|---|---|---|---|---|---|---|---|---|---|
| % | RRT | % | RRT | % | RRT | % | RRT | % | RRT |
| 0.04 | 0.85 | 0.23 | 0.86 | 0.50 | 0.88 | 0.49 | 0.88 | 2.33 | 0.85 |

Then, each amount of hydrazine produced in each product was obtained from the above results of measurement on the basis of the following assumptions.
1. The compound and Y-7 have almost the same absorbance. For example, if a ratio of the area of the peak indicating the production of Y-7 at the relative retention rate of 0.85 is 0.1%, 0.1% of the compound corresponds to Y-7. Therefore, if the compound weighs 50 mg, Y-7 weighs 0.5 mg.
2. The production of Y-7 from the compound progresses as an equimolar reaction, and the molecular weights of Y-7 and hydrazine are 306.75 and 32.05, respectively. Therefore, about 0.5 mg of Y-7 corresponds to about 0.05 mg of hydrazine.
3. This experiment is performed under assumption of intravenous drip infusion of 2 mg of the compound over 60 minutes per day for 5 days at the maximum.

The results are shown below. The amounts of hydrazine produced (µg) and the production rates (% (w/w)) of the hydrazine based on the active ingredient in the preparation examples are as follows.

**Table 7**

| An amount of the production of hydrazine | Preparation Example 17 | Preparation Example 18 | Preparation Example 19 | Preparation Example 20 | Preparation Example 21 |
|---|---|---|---|---|---|
| µg | 0.08 | 0.46 | 2 | 2 | 4.66 |
| % (w/w) | 0.004 | 0.023 | 0.05 | 0.049 | 0.23 |

The above results clearly indicate that the hydrazine production is remarkably suppressed in the products using a non-aqueous solvent.

### Industrial Applicability

According to the present invention, a novel pharmaceutical form of 6-[4-(4-pyridylamino)phenyl]-4,5-dihydro-3(2H)-pyridazinone useful as a cardiotonic agent can be provided.

Although the present invention has been explained in detail by referring to specific embodiments thereof, it is apparent to those skilled in the art that various alterations and modifications are possible without departing from the spirit and scope of the invention.

This application claims a conventional priority based on Japanese patent application filed on March 10, 2005 (Japanese Patent Application No. 2005-066521), and the entire disclosure thereof is incorporated herein by reference.

## Claims

1. Pharmaceutical products comprising 6-[4-(4-pyridylamino)phenyl]-4,5-dihydro-3(2H)-pyridazinone or a salt thereof, or a hydrate thereof or a solvate thereof as an active ingredient provided that an aqueous solution is excluded, wherein production of hydrazine is substantially suppressed.

2. The pharmaceutical products according to claim 1, wherein the active ingredient is 6-[4-(4-pyridylamino)phenyl]-4,5-dihydro-3(2H)-pyridazinone hydrochloride trihydrate.

3. The pharmaceutical products according to claim 1 or 2, wherein an amount of the hydrazine produced after storage at 60°C for 30 days is less than about 0.23% (w/w) based on the active ingredient.

4. The pharmaceutical products according to claim 1 or 2, wherein an amount of the hydrazine produced after storage at 60°C for 30 days is not more than about 0.05% (w/w) based on the active ingredient.

5. The pharmaceutical products according to any one of claims 1 to 4, wherein the active ingredient is dissolved in a non-aqueous solvent.

6. The pharmaceutical products according to claim 5, wherein the non-aqueous solvent consists of one or more types of solvents selected from glycerin, propylene glycol, polysorbate, ethanol and polyethylene glycol.

7. The pharmaceutical products according to any one of claims 1 to 4, which are lyophilized products.

8. The pharmaceutical products according to claim 7, which contain a saccharide.

9. The pharmaceutical products according to claim 7 or 8, which contain a pH stabilizer.

10. The pharmaceutical products according to any one of claims 7 to 9, which are dissolved upon use.

11. Pharmaceutical products containing 6-[4-(4-pyridylamino)phenyl]-4,5-dihydro-3(2H)-pyridazinone or a salt thereof, or a hydrate thereof or a solvate thereof as an active ingredient, wherein an amount of hydrazine produced after storage of the pharmaceutical products at 60°C for 30 days is less than about 0.23% (w/w) based on the active ingredient.

12. The pharmaceutical products according to claim 11, wherein the active ingredient is 6-[4-(4-pyridylamino)phenyl]-4,5-dihydro-3(2H)-pyridazinone hydrochloride trihydrate.

13. The pharmaceutical products according to claim 11 or 12, wherein the an amount of the hydrazine produced is not more than about 0.05% (w/w) based on the active ingredient.

14. The pharmaceutical products according to any one of claims 11 to 13, which are non-aqueous solution products, or lyophilized products.

15. The pharmaceutical products according to claim 14, wherein non-aqueous solvent of the non-aqueous solution products consist of one or more types of solvents selected from glycerin, propylene glycol, polysorbate, ethanol and polyethylene glycol.

16. The pharmaceutical products according to claim 14, wherein the lyophilized products contain a saccharide.

17. The pharmaceutical products according to claim 14 or 16, wherein the lyophilized products contain a pH stabilizer.

18. The pharmaceutical products according to any one of claims 14, 16 and 17, wherein the lyophilized products are dissolved upon use.

19. A method for stabilization of a solution containing 6-[4-(4-pyridylamino)phenyl]-4,5-dihydro-3(2H)-pyridazinone or a salt thereof, or a hydrate thereof or a solvate thereof, which comprises the step of adjusting a pH of the solution within a range of from about 3 to about 7.

20. The method for stabilization according to claim 19, wherein the solution contains a pH stabilizer.

21. The method for stabilization according to claim 19 or 20, wherein the solution contains an isotonic agent.
